# EUROPEAN PATENT SPECIFICATION

(11) **EP 3 191 210 B1**
(45) Date of publication and mention of the grant of the patent: **04.07.2018**
(21) Application number: 15762675.5
(22) Date of filing: 03.09.2015
(51) Int. Cl.: B01D 53/02, A61M 16/22

(54) **SORBENT MATERIAL**
SORBENSMATERIAL
MATÉRIAU SORBANT

(30) Priority: 12.09.2014 GB 201416154; 07.10.2014 GB 201417692
(43) Date of publication of application: 19.07.2017
(73) Proprietor: Johnson Matthey Public Limited Company, London EC4A 4AB (GB)
(72) Inventor: LUISMAN, Luke Alan, Reading Berkshire RG4 7EB (GB)
(74) Representative: Gleave, Robert James
(86) International application number: PCT/GB2015/052540
(87) International publication number: WO 2016/038339

(56) References cited:
- WO-A1-2011/049759
- DE-C1- 19 830 470
- US-A1- 2001 004 895
- US-A1- 2011 179 948
- US-A1- 2014 112 856

## Description

### Field of the Invention

The present invention relates to a process for removing carbon dioxide from a mixture of gases, and to a process for reducing the partial pressure of carbon dioxide in an enclosed space. The processes of the present invention employ a carbon dioxide adsorbent.

### Background

Removal of carbon dioxide from mixtures of gases is of interest in a number of fields. For example, it may be desirable to remove carbon dioxide from gases produced in industrial processes in order to reduce the carbon dioxide emissions associated with those processes. Carbon dioxide may be removed from mixtures of gases in enclosed spaces to reduce the partial pressure of carbon dioxide within those enclosed spaces (e.g. within a building). Carbon dioxide removal may also be desirable, for example, in breathing or ventilation apparatus, for example in medical applications, and for protection equipment for personnel exposed to hazardous environments.

Solid sorbent materials capable of adsorbing or absorbing carbon dioxide are of interest in carbon dioxide removal technologies. It is particularly advantageous to provide regenerable sorbent materials, as these permit removal of carbon dioxide from the sorbent material in order that it may be re-used.

US 6 279 576 describes a regenerative absorber device for the removal of carbon dioxide from expiration gases during anaesthesia. The device is provided with an ion exchanger having the capability to absorb carbon dioxide. This document proposes using ion exchange resins such as polystyrene-divinylbenzene copolymers with polyamine functional groups. Various options for regenerating the anion exchange resin are proposed, such as wet regeneration by passing an aqueous solution including a base such as sodium hydroxide over its absorbent bed, regeneration by heating, and regeneration by pressure reduction. The preferred regeneration method is to pass steam through the absorbent.

V. Zelenak et al (Reference 1) describe mesoprous silica with modified with 3-aminopropyl, 3-(methylamino) propyl and 3-(phenylaminopropyl) ligands, which are subjected to carbon dioxide sorption and desorption experiments. This document observes similar levels of carbon dioxide adsorption by the silica 3-aminopropyl sorbent (primary amine) and the silica 3-(methylamino) propyl sorbent (secondary amine). Lower adsorption was observed for silica 3-(phenylaminopropyl), indicating a weak interaction between the carbon dioxide and this material. The document proposes desorption using heating, or by purging with inert gas such as argon.

WO2011/049759 describes a process for removing carbon dioxide from a carbon dioxide containing gas stream using an ion exchange resin. A preferred resin is a polystyrene polymer based resin, which is cross-linked via the use of divinyl benzene, and is functionalised with primary amine groups including benylamine, wherein the resin is produced by the phthalimide process. This document discloses that it is necessary to increase the temperature of the resin to about 100°C in order to effect desorption of adsorbed carbon dioxide. US2001/0004895 discloses a breathing lime for anaesthesia apparatuses comprising a macroporous ion exchange resin with primary benzylamine groups. The absorbent can be regenerated by use of steam at a slightly increased pressure or under vacuum conditions or at a slightly raised temperature.

### Summary of the Invention

There remains a need for improved processes for the removal of carbon dioxide from mixtures of gases. In particular, there remains a need for sorbent materials for the removal of carbon dioxide, which provide excellent sorption of carbon dioxide and also are readily regenerated by desorption of the carbon dioxide. The present inventors have found that sorbent materials comprising benzyl amine moieties immobilised on a solid support exhibit excellent carbon dioxide sorption and permit thermal regeneration at a surprisingly low temperature. In particular, the examples below demonstrate that these materials exhibit high levels of carbon dioxide adsorption at room temperature, and yet can readily be regenerated by providing a flow of desorbing gas heated to approximately 60°C.

Accordingly, in a first preferred aspect, the present invention provides a process for removing carbon dioxide from a mixture of gases, the process comprising:
contacting the mixture of gases with a carbon dioxide adsorbent to adsorb carbon dioxide from the mixture of gases, wherein the adsorbent comprises benzyl amine moieties immobilised on a solid support; and
subsequently regenerating the adsorbent by desorbing carbon dioxide from the adsorbent, wherein the regeneration comprises heating to a temperature in the range from 55°C to 75°C and wherein the regeneration step is carried out at approximately atmospheric pressure.

Due to the excellent carbon dioxide adsorption at room temperature exhibited by these materials, in combination with regeneration requiring temperatures elevated only slightly above room temperature, the present inventors consider that their invention is particularly applicable to reducing carbon dioxide levels within enclosed spaces, in particular habitable spaces such as buildings, rooms, cabins or vehicles. Accordingly, in a second preferred aspect, the present invention provides a process for reducing the partial pressure of carbon dioxide in a mixture of gases in an enclosed space, the process comprising:
contacting at least portion of the mixture of gases from within the enclosed space with a carbon dioxide adsorbent to remove carbon dioxide from the mixture of gases, wherein the adsorbent comprises benzyl amine moieties immobilised on a solid support; and
subsequently regenerating the adsorbent by desorbing carbon dioxide from the adsorbent, wherein the regeneration comprises heating to a temperature in the range from 55°C to 75°C and wherein the regeneration step is carried out at approximately atmospheric pressure.
The adsorbent may be located within the enclosed space. Alternatively, a portion of the mixture of gases from within the enclosed space may be removed from the enclosed space, contacted with the adsorbent and then the treated mixture of gases returned to the enclosed space. For example, the portion of the mixture of gases may be flowed over or through a bed of adsorbent.

The nature of the enclosed space is not particularly limited in the present invention. For example, the enclosed space may be a room, a building, an underground space such as a mine, or a cabin or other habitable area of a vehicle (e.g. a boat, aircraft, road vehicle, train etc.). As used herein, the term enclosed space is intended to include areas where gas exchange with the area outside the space is restricted to an extent. The enclosed space need not be completely sealed or isolated from the area outside the enclosed space.

### Brief Description of the Drawings

**Figure 1** shows a plot of TCD signal and M/Z 44 mass spec signal against temperature for carbon dioxide desorption from Quadrapure BZA in a flow of Argon as determined in Example 2.

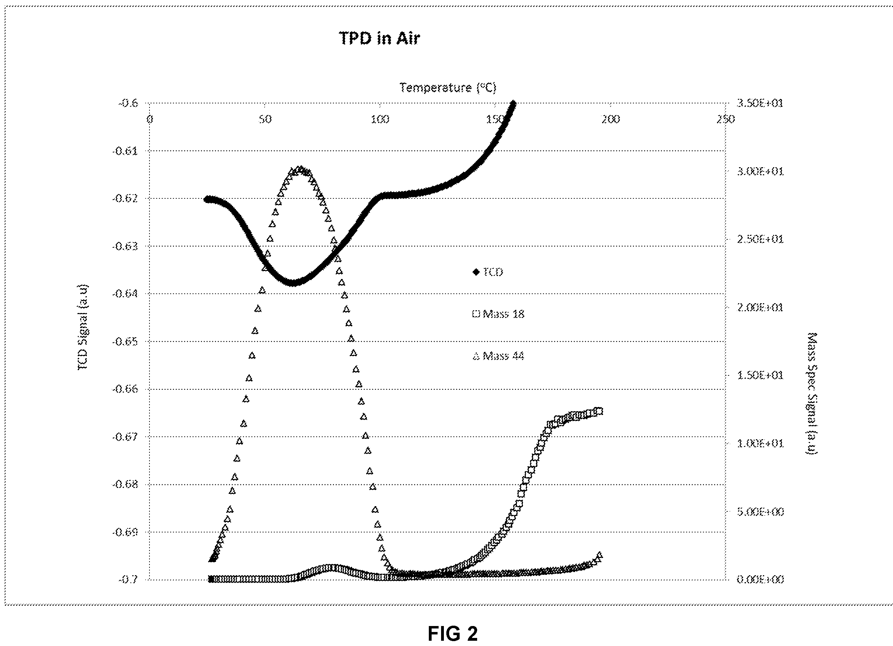

**Figure 2** shows a plot of TCD signal and M/Z 44 mass spec signal against temperature for carbon dioxide desorption from Quadrapure BZA in a flow of air as determined in Example 2.

### Detailed Description

Preferred and/or optional features of the invention will now be set out. Any aspect of the invention may be combined with any other aspect of the invention, unless the context demands otherwise. Any of the preferred or optional features of any aspect may be combined, singly or in combination, with any aspect of the invention, unless the context demands otherwise.

The adsorbent materials of the present invention comprise a benzyl amine moiety. The amine group of the benzyl amine moiety may be a primary amine, a secondary amine or a tertiary amine. Preferably, the amine group of the benzyl amine moiety is a primary amine or a secondary amine, most preferably a primary amine.

As the skilled person will understand, the benzyl amine moiety may have the following formula: 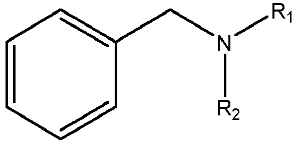 wherein R₁ and R₂ are each independently H or optionally substituted C₁-C₁₀ (e.g. C₁-C₅ or C₁-C₃) hydrocarbon group. Preferably, at least one of R₁ and R₂ is H. More preferably, both R₁ and R₂ are H.

In some embodiments, it is preferable that R₁ is unsubstituted. In some embodiments, it is preferable that R₂ is unsubstituted.

As used herein, the term optionally substituted includes moieties in which a one, two, three, four or more hydrogen atoms have been replaced with other functional groups. Suitable functional groups include -OH, -SH, -OR₃, -SR₃, -NR₃R₃, C(O)COR₃, -OC(O)R₃, -NR₃C(O)R₃ and C(O)NR₃R₃, wherein each R₃ is independently H or C₁ to C₁₀ (e.g. C₁-C₅ or C₁-C₃) alkyl or alkenyl, preferably alkyl. For example, suitable substituent functional groups include -OH, -OR₃, -NR₃R₃, C(O)COR₃, -OC(O)R₃, -NR₃C(O)R₃ and C(O)NR₃R₃, wherein each R₃ is independently H or C₁ to C₁₀ (e.g. C₁-C₅ or C₁-C₃) alkyl or alkenyl, preferably alkyl.

As used herein, the term hydrocarbon moiety is intended to include alkyl (including cycloalkyl), alkenyl, alkynyl, aryl and alkaryl. The hydrocarbon moiety may be linear or branched. It is preferable that the hydrocarbon moiety is alkyl or alkenyl, more preferably alkyl, which may be linear or branched.

The benzyl amine moiety is immobilised on a solid support. It may be preferred that the benzyl amine moiety is covalently attached to the solid support. This can help to improve the stability of the adsorbent through multiple adsorption and desorption cycles, as covalent attachment tends to be stronger than other forms of immobilisation. Where the benzyl amine moiety is covalently attached to the solid support, the point of attachment of the benzyl amine moiety is not particularly limited, but preferably it is via one of the ring carbon atoms of the benzene ring of the benzyl amine moiety.

It will be understood that in the processes of the present invention the steps of contacting the adsorbent with a mixture of gases and subsequently regenerating the adsorbent may be repeated one or more times over the lifetime of the adsorbent material. For example, the steps may be repeated two or more times, 5 or more times, or 10 or more times.

The solid support may be a polymeric solid support. The benzyl amine moiety may be covalently attached to the polymer backbone of the polymer of a polymeric solid support. In a particularly preferred embodiment, the solid support is a polystyrene solid support, and the benzyl amine moiety is covalently attached to the polystyrene polymer.

The nature of the solid support is not particularly limited in the present invention. It may be preferable that it is a polymeric support. It may be preferable that it is porous. Suitable polymeric supports include poly alkene polymers, such as polyvinyl polymers. A particularly suitable poly vinyl polymer is polystyrene. Poly(vinyltoluene), poly(ethylstyrene), poly(alpha-methyl styrene), poly(chlorostyrene) and poly(chloromethylstyrene) may also be suitable. The polymer of the polymeric solid support may be cross-linked, for example using cross linkers such as divinyl aliphatic or aromatic compounds. Silica solid supports, including porous silica solid supports may also be suitable.

The solid support is typically a particulate solid support, although this is not essential. The particles size is not particularly limited. Typical particle diameters are in the range from 450µm to 600µm. For example, the particles may have a d50 particle diameter of at least 50µm, at least 100µm, at least 200µm, or at least 300µm. For example, the particles may have a d₅₀ particle diameter of 5mm or less, 2mm or less, 1mm or less, 900µm or less, or 800µm or less.

In the processes of the present invention, a mixture of gases containing carbon dioxide is contacted with adsorbent material. The adsorbent material adsorbs carbon dioxide from the mixture of gases, thereby removing some or all of the carbon dioxide from the mixture of gases. The mixture of gases may preferably be contacted with the adsorbent by flowing the mixture over or through a bed of adsorbent.

As demonstrated in the examples, high levels of adsorption are achieved at approximately room temperature. This is particularly convenient, as it permits the materials to be used for carbon dioxide removal from a mixture of gases without substantially altering the temperature of the mixture of gases, for example in air purification applications in buildings or vehicles. Accordingly, it may be preferred that the step of contacting the adsorbent with the mixture of gases is carried out at approximately ambient temperature. For example, the step of contacting the adsorbent with the mixture of gases may advantageously be carried out at a temperature of 40°C or less, e.g. 35°C or less or 30°C or less. The minimum temperature is not particularly limited in the present invention, but may for example be at least -20°C, at least -10°C, at least 0°C, at least 10°C or at least 15°C.

The mixture of gases may be humidified before it is contacted with the adsorbent. The present inventors have found that this can enhance carbon dioxide adsorption.

The present inventors have found that the adsorbent is suitable for removing carbon dioxide from mixtures of gases comprising relatively low concentrations of carbon dioxide, such as air. Carbon xiodie is typically present in air at a concentration of about 400 ppm. Under "stuffy" conditions, carbon dioxide concentration may rise to levels of 1000 to 5000 ppm. The mixture of gases may include 10000 ppm or less carbon dioxide, preferably 8000 ppm or less, 600 ppm or less, 5000 ppm or less, 3000 ppm or less, 2000 ppm or less, 1500 ppm or less or 1000 ppm or less carbon dioxide, for example. The minimum concentration of carbon dioxide is not particularly limited, but it may be, for example, at least 50 ppm, at least 100 ppm or at least 200 ppm.

The adsorbent is regenerated by heating to a temperature in the range from 55°C to 75°C. It is preferable that the desorption temperature is significantly higher than is necessary to effect desorption to avoid wasting energy. Accordingly, it may be preferable that the adsorbent is regenerated by heating to a temperature of 70°C or less, or 65°C or less. A particularly suitable range is 55°C to 65°C.

The regeneration step may additionally comprise supplying a desorbing gas flow to the adsorbent material. The nature of the desorbing gas flow is not particularly limited. It may be an inert gas such as argon, or nitrogen. Conveniently, the desorbing gas flow may be a mixture of gases, such as air. The desorbing gas flow may even include carbon dioxide and still provide satisfactory desorption, where desorption takes place in the temperature range from 40°C to 75°C. Where carbon dioxide is removed from a mixture of gases from within an enclosed space, preferably the desorbing gas flow is expelled at a position external to the enclosed space.

The regeneration step is carried out at approximately atmospheric pressure.

The step of contacting the mixture of gases with a carbon dioxide adsorbent may take place at approximately atmospheric pressure, or at pressures above or below atmospheric pressure. However, atmospheric pressure is preferred as this is typically more convenient.

### Examples

### Example 1 - Carbon Dioxide Sorption using Quadrapure BZA

Carbon dioxide adsorption and desorption using an adsorbent comprising macroporous polystyrene support with covalently immobilised primary benzyl amine groups was examined. Suitable benzylamine functionalised macroporous polystyrene can be obtained from Alfa Aesar (UK) under the brand name Quadrapure BZA®.

0.5187g of Quadrapure BZA was used in the absorption experiments. The sample was heated under flowing nitrogen (100 ml/min) for 1 hour to desorb CO₂ and H₂O from absorbed ambient air. The sample was then allowed to cool to room temperature. 5000ppm CO₂ in air (1000 ml/min) was flowed over the sample, and the amount of CO₂ absorbed was monitored using an IR Multigas analyser. Flow continued until the detected CO₂ returned to baseline levels (indicating saturation of the Quadrapure BZA sample). The Quadrapure BZA sample absorbed 5.1 wt% CO₂ (corresponding to approximately 51 mg of CO₂ per g of sample).

The experiment was repeated to probe wet absorption. The protocol above was followed, except that the 5000ppm CO₂ in air (1000 ml/min) was flowed through a trough bubbler containing water held at 30°C before contact with the sample. Under wet conditions, the Quadrapure BZA sample absorbed 6.3 wt% CO₂ (corresponding to approximately 63 mg of CO₂ per g of sample).

### Example 2 - Carbon Dioxide Desorption from Quadrapure BZA

Temperature programmed carbon dioxide desorption was used to determine the temperature at which carbon dioxide is desorbed from Quadrapure BZA.

Quadrapure BZA was pre-treated by heating under dry argon for 30 minutes, then cooled to 25°C and contacted with a gas flow on 15% carbon dioxide in nitrogen for 30 minutes to saturate the sample with carbon dioxide. The gas flow was switched to argon and the sample gradually heated to 200°. The carbon dioxide levels in the argon flow after contact with the Quadrapure BZA was monitored as the temperature changed using TCD (thermal conductivity detector). Mass spectrometry was also carried out to detect carbon dioxide in the argon flow after contact with the Quadrapure BZA.

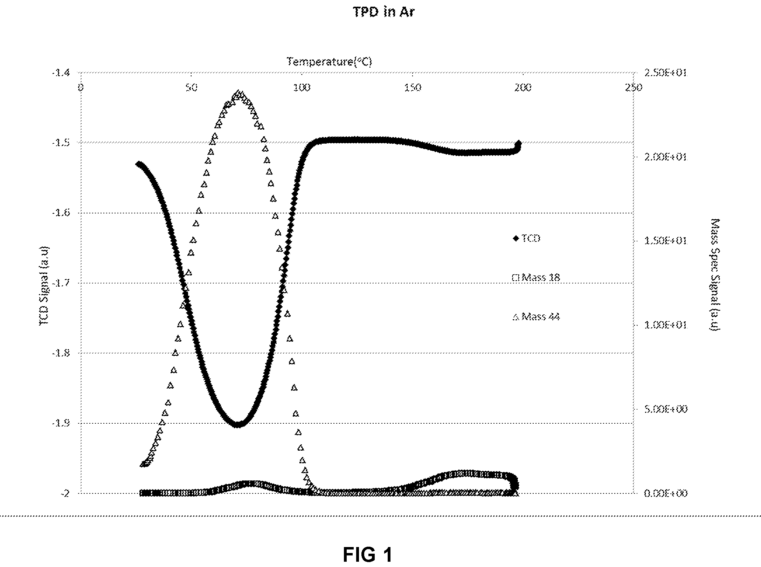

Figure 1 shows the plot of TCD signal and M/Z 44 mass spectroscopy signal against temperature. The signals closely follow each other, and illustrate that carbon dioxide is desorbed at about 60°C.

### References

1. V. Zelenak et al, Microporous and Mesoprous Materials 116 (2008) 358-364

## Claims

1. A process for removing carbon dioxide from a mixture of gases, the process comprising:
contacting the mixture of gases with a carbon dioxide adsorbent to adsorb carbon dioxide from the mixture of gases, wherein the adsorbent comprises benzyl amine moieties immobilised on a solid support; and
subsequently regenerating the adsorbent by desorbing carbon dioxide from the adsorbent, wherein the regeneration comprises heating to a temperature in the range from 55°C to 75°C and wherein the regeneration step is carried out at approximately atmospheric pressure.

2. A process according to claim 1 wherein the amine group of the benzyl amine moiety is a primary amine.

3. A process according to any one of the preceding claims wherein the regeneration step comprises supplying a desorbing gas flow to the adsorbent.

4. A process according to claim 3 wherein the desorbing gas is air.

5. A process according to any one of the preceding claims wherein the benzyl amine moiety is covalently attached to the solid support.

6. A process according to any one of the preceding claims wherein the solid support is a poly(alkene) solid support.

7. A process according to claim 6 wherein the solid support is a polystyrene support.

8. A process according to any one of the preceding claims wherein the adsorbent comprises primary benzyl amine covalently attached to a polystyrene solid support.

9. A process according to any one of the preceding claims wherein the mixture of gases is humidified before it is contacted with the adsorbent.

10. A process according to any one of the preceding claims which is a process for reducing the partial pressure of carbon dioxide a mixture of gases in an enclosed space.

11. A process according to claim 10 wherein the method comprises contacting at least a portion of the mixture of gases from within the enclosed space with the carbon dioxide adsorbent to remove carbon dioxide from the mixture of gases, and returning the treated mixture of gases to the enclosed space.

12. A process according to claim 10 or claim 11 wherein the enclosed space is selected from a room; a building; an underground space such as a mine; and a cabin or other habitable area of a vehicle.

13. A process for reducing the partial pressure of carbon dioxide in a mixture of gases in an enclosed space, the process comprising:
contacting at least portion of the mixture of gases from within the enclosed space with a carbon dioxide adsorbent to remove carbon dioxide from the mixture of gases, and returning the treated mixture of gases to the enclosed space, wherein the adsorbent comprises benzyl amine moieties immobilised on a solid support; and
subsequently regenerating the adsorbent by desorbing carbon dioxide from the adsorbent, wherein the regeneration comprises heating to a temperature in the range from 55°C to 75°C and wherein the regeneration step is carried out at approximately atmospheric pressure.

## Patentansprüche

1. Verfahren zum Entfernen von Kohlendioxid aus einem Gasgemisch, wobei das Verfahren umfasst:
Inkontaktbringen des Gasgemischs mit einem Kohlendioxid-Adsorptionsmittel, um Kohlendioxid aus dem Gasgemisch zu adsorbieren, wobei das Adsorptionsmittel auf einem festen Träger immobilisierte Benzylaminteile umfasst; und
anschließendes Regenerieren des Adsorptionsmittels durch Desorbieren von Kohlendioxid aus dem Adsorptionsmittel, wobei das Regenerieren das Erhitzen auf eine Temperatur im Bereich von 55 °C bis 75 °C umfasst und der Regenerierungsschritt bei etwa Atmosphärendruck durchgeführt wird.

2. Verfahren nach Anspruch 1, wobei die Amingruppe des Benzylaminteils primäres Amin ist.

3. Verfahren nach einem der vorhergehenden Ansprüche, wobei der Regenerierungsschritt das Zuführen eines desorbierenden Gasstroms zum Adsorptionsmittel umfasst.

4. Verfahren nach Anspruch 3, wobei das desorbierende Gas Luft ist.

5. Verfahren nach einem der vorhergehenden Ansprüche, wobei der Benzylaminteil kovalent an den festen Träger gebunden ist.

6. Verfahren nach einem der vorhergehenden Ansprüche, wobei der feste Träger aus Poly(alken) ist.

7. Verfahren nach Anspruch 6, wobei der feste Träger aus Polystyrol ist.

8. Verfahren nach einem der vorhergehenden Ansprüche, wobei das Adsorptionsmittel primäres Benzylamin umfasst, das kovalent an einen festen Polystyrolträger gebunden ist.

9. Verfahren nach einem der vorhergehenden Ansprüche, wobei das Gasgemisch befeuchtet wird, bevor es mit dem Adsorptionsmittel in Kontakt gebracht wird.

10. Verfahren nach einem der vorhergehenden Ansprüche, das ein Verfahren zur Verringerung des Partialdrucks von Kohlendioxid in einem Gasgemisch in einem geschlossenen Raum ist.

11. Verfahren nach Anspruch 10, wobei das Verfahren das Inkontaktbringen mindestens eines Teils des Gasgemischs aus dem geschlossenen Raum mit dem Kohlendioxid-Adsorptionsmittel, um Kohlendioxid aus dem Gasgemisch zu entfernen, und das Rückführen des behandelten Gasgemischs in den geschlossenen Raum umfasst.

12. Verfahren nach Anspruch 10 oder Anspruch 11, wobei der geschlossene Raum aus einem Zimmer; einem Gebäude; einem unterirdischen Raum wie einer Mine; und einer Kabine oder einem anderen bewohnbaren Bereich eines Fahrzeugs ausgewählt wird.

13. Verfahren zur Verringerung des Partialdrucks von Kohlendioxid in einem Gasgemisch in einem geschlossenen Raum, wobei das Verfahren umfasst:
Inkontaktbringen mindestens eines Teils des Gasgemischs aus dem geschlossenen Raum mit einem Kohlendioxid-Adsorptionsmittel, um Kohlendioxid aus dem Gasgemisch zu entfernen, und Rückführen des behandelten Gasgemischs in den geschlossenen Raum, wobei das Adsorptionsmittel auf einem festen Träger immobilisierte Benzylaminteile umfasst; und
anschließendes Regenerieren des Adsorptionsmittels durch Desorbieren von Kohlendioxid aus dem Adsorptionsmittel, wobei das Regenerieren das Erhitzen auf eine Temperatur im Bereich von 55 °C bis 75 °C umfasst und der Regenerierungsschritt bei etwa Atmosphärendruck durchgeführt wird.

## Revendications

1. Procédé pour éliminer le dioxyde de carbone d'un mélange de gaz, le procédé comprenant :
la mise en contact du mélange de gaz avec un adsorbant de dioxyde de carbone pour adsorber le dioxyde de carbone à partir du mélange de gaz, l'adsorbant comprenant des groupements benzylamine immobilisés sur un support solide ; et
la régénération ultérieure de l'adsorbant par désorption du dioxyde de carbone de l'adsorbant, où la régénération comprend un chauffage à une température comprise entre 55 °C et 75 °C et où l'étape de régénération est effectuée approximativement à la pression atmosphérique.

2. Procédé selon la revendication 1 dans lequel le groupe amine du groupement benzylamine est une amine primaire.

3. Procédé selon l'une quelconque des revendications précédentes dans lequel l'étape de régénération comprend l'apport d'un flux de gaz désorbant sur l'adsorbant.

4. Procédé selon la revendication 3 dans lequel le gaz désorbant est de l'air.

5. Procédé selon l'une quelconque des revendications précédentes dans lequel le groupement benzylamine est fixé par covalence au support solide.

6. Procédé selon l'une quelconque des revendications précédentes dans lequel le support solide est un support solide de poly(alcène).

7. Procédé selon la revendication 6 dans lequel le support solide est un support de polystyrène.

8. Procédé selon l'une quelconque des revendications précédentes dans lequel l'adsorbant comprend une benzylamine primaire fixée par covalence à un support solide de polystyrène.

9. Procédé selon l'une quelconque des revendications précédentes dans lequel le mélange de gaz est humidifié avant d'être mis en contact avec l'adsorbant.

10. Procédé selon l'une quelconque des revendications précédentes qui est un procédé pour diminuer la pression partielle de dioxyde de carbone dans un mélange de gaz dans un espace clos.

11. Procédé selon la revendication 10, lequel procédé comprend la mise en contact d'au moins une partie du mélange de gaz depuis l'intérieur de l'espace clos avec l'adsorbant de dioxyde de carbone pour éliminer le dioxyde de carbone du mélange de gaz, et le renvoi dans l'espace clos du mélange de gaz ainsi traité.

12. Procédé selon la revendication 10 ou la revendication 11 dans lequel l'espace clos est sélectionné parmi une pièce ; un bâtiment ; un espace souterrain tel qu'une mine ; et un habitacle ou une autre zone habitable d'un véhicule.

13. Procédé pour diminuer la pression partielle de dioxyde de carbone dans un mélange de gaz dans un espace clos, le procédé comprenant :
la mise en contact d'au moins une partie du mélange de gaz depuis l'intérieur de l'espace clos avec un adsorbant de dioxyde de carbone pour éliminer le dioxyde de carbone du mélange de gaz, et le renvoi dans l'espace clos du mélange de gaz ainsi traité, l'adsorbant comprenant des groupements benzylamine immobilisés sur un support solide ; et
la régénération ultérieure de l'adsorbant par désorption du dioxyde de carbone de l'adsorbant, où la régénération comprend un chauffage à une température comprise entre 55 °C et 75 °C et où l'étape de régénération est effectuée approximativement à la pression atmosphérique.
